(19) Europäisches Patentamt
European Patent Office
Office européen des brevets

(11) Numéro de publication : **0 454 588 A1**

(12) # DEMANDE DE BREVET EUROPEEN

(21) Numéro de dépôt : **91401113.5**

(51) Int. Cl.$^5$ : **C07C 47/21**, C07C 45/51

(22) Date de dépôt : **26.04.91**

(30) Priorité : **27.04.90 FR 9005380**

(43) Date de publication de la demande :
**30.10.91 Bulletin 91/44**

(84) Etats contractants désignés :
**AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Jacquot, Roland**
**La Dovay, 97 Chemin de la Courtille**
**F-69110 Sainte Foy Les Lyon (FR)**
Inventeur : **Mercier, Claude**
**85 avenue du Point du Jour**
**F-69005 Lyon (FR)**

(74) Mandataire : **Savina, Jacques et al**
**RHONE-POULENC RORER S.A., Direction des Brevets, 20 avenue Raymond Aron**
**F-92160 Antony Cédex (FR)**

(54) **Procédé de préparation de citral.**

(57) La présente invention concerne un procédé de préparation du citral par craquage de l'acetal diprénylique du prénal en phase vapeur en présence d'un catalyseur de type hétérogène acide.

EP 0 454 588 A1

La présente invention concerne un procédé de préparation du citral. Plus particulièrement, elle concerne un procédé de préparation de citral par craquage de l'acétal diprénylique du prénal en phase vapeur.

Le citral ainsi obtenu constitue un intermédiaire dans la synthèse de la vitamine A, et peut donc être utilisé à ce titre. Il peut également être utilisé pour ses propriétés aromatiques.

Il est connu dans l'art antérieur de préparer le citral par craquage de l'acétal diprénylique du prénal en phase liquide.

En particulier, il est connu d'après le brevet français n° 72.40678 publié sous le numéro 2.160.125, de préparer des aldéhydes à liaisons α,β-éthyléniques par réaction à température élevée en phase liquide, d'un aldéhyde à liaison α,β-éthylénique et d'un alcool allylique. L'aldéhyde à liaison α,β-éthylénique peut être la diméthyl-3,3 acroléine connue sous le nom de prénal, et l'alcool éthylénique peut être le méthyl-3 butène-2 ol-1 connu sous la dénomination de prénol.

La réaction de condensation est réalisée de préférence en présence d'un catalyseur acide à des températures comprises entre 100 et 250 °C. L'acide utilisé comme catalyseur est choisi parmi les acides non organiques tels que sulfuriques, phosphoriques, halogénés, nitriques, sulfureux, phosphoreux, perchlorique, borique, silicique, les sels d'acides à hydrogène dissociable. On peut aussi utiliser les acides organiques. La quantité d'acide utilisée lorsqu'il s'agit d'un acide minéral présentant un pK de 0 à environ 3 est comprise entre 0,01 et 0,5 %. D'autres brevets, tel le brevet français FR 1.582.515 décrivent des variantes et des perfectionnements à ce type de procédés.

Il est encore connu d'après le brevet américain 4.288.636 de préparer le citral par chauffage d'un acétal diprénylique en présence d'acide phosphorique et d'un liquide inerte ayant, à la pression de réaction, un point d'ébullition supérieur au prénol et inférieur au citral. Le liquide inerte est de préférence le 3,3,7-triméthyl-4 oxa octa-1,6-diène.

La quantité d'acide phosphorique utilisée est comprise entre 0,001 et 0,5 % en poids, et de préférence, entre 0,005 et 0,05 % en poids.

Cependant, ces procédés de préparation en phase liquide présentent de nombreux inconvénients. En particulier, en raison des volumes très importants de liquides à manipuler, de la difficulté, voire de l'impossibilité, de réaliser des réactions en continu, et également du coût important de ces opérations, ces procédés sont difficiles à exploiter au niveau industriel. Pour remédier à ces inconvénients, la présente invention a pour objet un procédé de préparation du citral par craquage d'acétal diprénylique en phase vapeur, en présence d'un catalyseur de type hétérogène acide.

L'un des points essentiels de l'invention réside dans le choix du catalyseur, qui est déterminant dans l'efficacité de la réaction. En effet, nous avons constaté qu'en présence de catalyseurs présentant des acidités trop élevées, le citral était transformé en produits secondaires de cyclisation, tel le para cymène. Cette réaction de dégradation du citral avait déjà été observée par Clark et coll.; Tetrahedron, vol 33 p2187, (1977); qui ont identifié 14 composés de cyclisation obtenus en milieu acide à partir du citral. Plus précisément, ces auteurs ont étudié les produits de dégradation du citral en présence d'acide chlorhydrique, à des pH compris entre 2,4 et 3,2. Ils ont ainsi mis en évidence des produits nouveaux, et proposé un schéma réactionnel. Cependant, il est clair que la liste des produits obtenus n'est pas exhaustive, et que des produits non encore identifiés doivent se former.

Afin d'obtenir des taux importants de citral, il est donc nécessaire d'effectuer la réaction en présence de catalyseurs présentant des acidités suffisamment élevées pour réaliser le craquage de l'acétal, mais pas trop élevées pour ne pas transformer le citral en produits secondaires. Dans ces conditions, les catalyseurs utilisés sont des catalyseurs acides de type hétérogène, sélectionnés en fonction de leur comportement dans une réaction test de transformation du citral en produits de cyclisation.

Cette réaction consiste à introduire, dans un réacteur 1 ml de catalyseur entre deux lits de 5 ml de quartz. On active ensuite le catalyseur par chauffage à 300°C pendant une heure sous un courant d'azote de 3 litres par heure. Enfin, on injecte, pendant une heure, le citral à un débit de 4 ml par heure, tout en condensant le gaz issu de la réaction. Le condensat ainsi obtenu est analysé par chromatographie en phase gazeuse.

Les catalyseurs ayant la bonne acidité, utilisables dans la présente invention, ne transforment pas, dans les conditions définies ci-dessus, plus de 30 % du citral en produits secondaires.

La présente invention a donc pour objet un procédé de préparation de citral par craquage d'acétal diprénylique du prénal, caractérisé en ce que ledit craquage est effectué en phase vapeur, en présence d'un catalyseur de type hétérogène acide qui, placé pendant 1 heure dans un réacteur, à raison de 1 ml entre deux lits de 5 ml de quartz, en présence d'un courant de citral de 4 ml/h, sous un courant d'azote à 3 l/h et à une température de 300°C, ne transforme pas plus de 30 % de citral en produits secondaires.

La réaction peut être schématisée de la manière suivante:

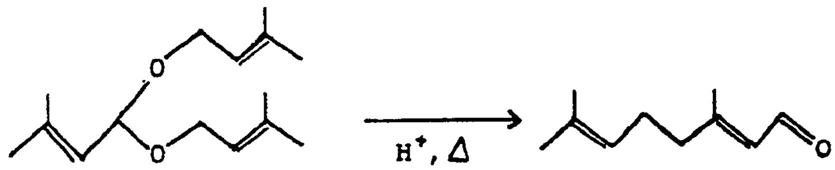


Acétal Citral

Dans un mode de réalisation de la présente invention, on utilise des catalyseurs possèdant une capacité d'échange de cations, tels qu'en particulier les zéolites.

On peut utiliser à ce sujet les zéolites du commerce, auxquelles, le cas échéant, certaines modifications sont apportées. Préférentiellement, on utilise le tamis moléculaire 3Å, ou la mordénite échangée au sodium.

Dans un autre mode de réalisation, on peut utiliser comme catalyseur des oxydes de métaux appartenant aux colonnes 3b à 6b de la classification periodique des élements. Les oxydes préférés sont en particulier les oxydes de zirconium, molybdène, vanadium, ou cérium.

Toujours selon la présente invention, les catalyseurs hétérogènes acides peuvent être des sels, tels qu'en particulier les phosphates, les silicates, ou les chromites. De preference, on utilise le silicate de calcium, le phosphate de lithium, ou le chromite de cuivre.

Le procédé de craquage d'acétal selon la présente invention est réalisé à une température comprise entre la température d'ébullition de l'acétal diprénylique du prénal, c'est-à-dire 230°C environ, et 400°C environ, et de preference, entre 250°C et 350°C. En outre, on peut opérer sous pression réduite ou à la pression atmosphérique. Il permet d'obtenir de très bon rendements en citral.

Une manière pratique de mettre en oeuvre la présente invention consiste à introduire dans un réacteur une quantité désirée de catalyseur, eventuellement entre deux lits de quartz pour favoriser la mise en contact des réactifs. La température du réacteur est ensuite élevée jusqu'à une valeur déterminée, comprise entre la température d'ébullition de l'acetal et 400°C environ, de manière à activer le catalyseur. On injecte ensuite , au débit souhaité,l'acétal en présence d'un gaz inerte tel que l'hélium, l'argon ou l'azote, on condense les gaz, et on analyse les produits.

Avantageusement, pour 1 ml de catalyseur, le gaz inerte est injecté à un débit compris entre 0,1 et 10 litres par heure, et l'acetal, à un débit liquide compris entre 0 et 10 ml par heure, et de préférence, entre 0 et 5 ml par heure.

La présente invention sera plus complètement décrite à l'aide des exemples suivants, qui doivent être considérés comme illustratifs et non limitatifs.

## EXEMPLE 1

Test d'acidité de catalyseurs.

Dans un réacteur cylindrique en verre de 18 mm de diamètre, on introduit 1 ml de catalyseur entre deux lits de 5 ml de quartz. On active ce catalyseur à 300°C pendant une heure, sous courant d'azote à 3 litres par heure. Le chauffage est assuré par un four électrique. On injecte ensuite le citral à 4 ml par heure, pendant une heure, à l'aide d'un pousse seringue. Le gaz est condensé, et on analyse le condensat par chromatographie en phase gazeuse.

TT = Taux de Transformation : correspond au nombre de moles de citral présentes au départ, moins le nombre de moles de citral restant à l'arrivée, divisées par le nombre de moles de citral présentes au départ.

RR = Rendement Réel : correspond au nombre de moles obtenues sur le nombre de moles engagées.

O = Catalyseur dont l'acidité correspond aux exigences du procédé de l'invention.

N = Catalyseur ayant une acidité trop élevée pour le procédé de l'invention

| Catalyseur | TT % citral | RR% pcymene | cyclisé autre | Acidité O/N |
|---|---|---|---|---|
| Phosphate de Lithium | 14 | 0-1 | 10 | O |
| Tamis moléculaire 3Å | 22 | 0-1 | 20 | O |
| ZrO2 hydratée | 13 | 0-1 | 12 | O |
| Mordénite $Na^+$ | 28 | 0-1 | 21 | O |
| Mordénite H | 54 | 14 | 21 | N |
| Chromite de Cu | 20 | 0-1 | 12 | O |
| ZM 980 Zeocat | 81 | 26 | 18 | N |
| HZSM 5 | 47 | 6 | 14 | N |

Les catalyseurs transformant moins de 30% du citral introduit en produits secondaires correspondent à l'acidité telle que définie dans le procédé selon l'invention. En revanche, les autres catalyseurs acides de type hétérogène qui transforment plus de 30% du citral en produits secondaires ne peuvent être utilisés dans le procédé.

EXEMPLE 2

Dans un réacteur tubulaire en verre de 18 mm de diamètre, on dispose 1 ml de catalyseur entre deux lits de 5 ml de quartz. On préchauffe le lit catalytique 1 heure à 350°C sous courant d'azote 8 litres par heure. Au moyen d'un pousse seringue, on injecte l'acetal diprénylique du prénal à un débit liquide de 2 ml par heure. Après une heure de réaction en phase vapeur, l'analyse du condensat par chromatographie en phase gazeuse donne le TT en acétal et le RR en citral.

| Catalyseur | TT % acetal | RR % citral |
|---|---|---|
| Mordénite $Na^+$ | 100 | 30 |
| Tamis molec. 3Å | 80 | 30 |
| Oxyde de vanadium | 84 | 22 |
| Phosphate de lithium | 96 | 30 |
| Silicate de calcium | 95 | 27 |
| Chromite de cuivre | 100 | 24 |
| Zircone | 100 | 30 |
| Oxyde de molybdene | 80 | 25 |
| Oxyde de cérium | 95 | 25 |
| $ZrO_2$ + Cr | 75 | 22 |

EXEMPLE 3

Dans un réacteur tubulaire en verre de 18 mm de diamètre, on dispose 10 ml de catalyseur. On préchauffe le lit catalytique à 250°C sous courant d'azote 0,6 litre par heure. Au moyen d'un pousse seringue, on injecte l'acetal diprénylique du prénal à un débit liquide de 2 ml par heure. Après une heure de réaction en phase gazeuse, l'analyse du condensat par chromatographie en phase gazeuse donne le TT en acetal et le RR en citral.

| Catalyseur | TT % acetal | RR % citral |
|---|---|---|
| Tamis 3Å | 100 | 85 |
| Mordénite $Na^+$ | 85 | 36 |
| Zircone | 100 | 54 |

**Revendications**

1. Procédé de préparation de citral par craquage de l'acétal diprénylique du prénal caractérisé en ce que ledit craquage est effectué en phase vapeur, en présence d'un catalyseur de type hétérogène acide qui, placé pendant 1 heure dans un réacteur, à raison de 1 ml entre deux lits de 5 ml de quartz, en présence d'un courant de citral de 4 ml/h, sous un courant d'azote de 3 l/h et à une température de 300°C, ne transforme pas plus de 30 % de citral en produits secondaires.

2. Procédé selon la revendication 1 caractérisé en ce que le catalyseur de type hétérogène acide est une zéolite.

3. Procédé selon la revendication 2 caractérisé en ce que la zéolite est choisie parmi la Mordénite échangée au sodium, ou le tamis moléculaire 3Å.

4. Procédé selon la revendication 1 caractérisé en ce que le catalyseur de type hétérogène acide est un oxyde d'un metal appartenant aux colonnes 3b à 6b de la classification périodique des élements.

5. Procédé selon la revendication 4 caractérisé en ce que l'oxyde est choisi parmi les oxydes de zirconium, de Molybdène, de Vanadium, et de Cérium.

6. Procédé selon la revendication 1 caractérisé en ce que le catalyseur de type hétérogène acide est un sel, choisi de préference parmi le silicate de calcium, le phosphate de lithium, ou le chromite de cuivre.

7. Procédé selon l'une des revendications précedentes caractérisé en ce qu'il est effectué à une température comprise entre la température d'ébullition de l'acetal diprénylique du prénal et 400°C environ, et de preference, entre 250°C et 350°C.

8. Procédé selon l'une des revendications précedentes caractérisé en ce qu'il est effectué à une pression inférieure ou égale à la pression atmosphérique.

9. Procédé selon l'une des revendications précedentes caractérisé en ce que, pour 1 ml de catalyseur, l'acétal diprénylique du prénal est introduit à un débit liquide compris entre 0 et 10 ml par heure, et de préférence, entre 0 et 5 ml par heure.

10. Procédé selon l'une des revendications précedentes caractérisé en ce que, pour 1 ml de catalyseur, le gaz inerte est introduit à un débit compris entre 0,1 et 10 litres par heure.

Office européen des brevets

# RAPPORT DE RECHERCHE EUROPEENNE

Numero de la demande

EP 91 40 1113

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | DE-A-2411530 (HOFFMANN-LA_ROCHE)<br>* page 7, alinéa 2 *<br>--- | 1, 6-8 | C07C47/21<br>C07C45/51 |
| A | EP-A-287956 (BASF)<br>* page 4, ligne 21 - page 4, ligne 24 *<br>--- | 1, 4 | |
| A | EP-A-10235 (RUHRCHEMIE)<br>* page 4, ligne 24 - page 5, ligne 2 *<br>--- | 1 | |
| A | PATENT ABSTRACTS OF JAPAN<br>vol. 12, no. 382 (C-535)(3229) 12 octobre 1988,<br>& JP-A-63 130551 (KAWAKEN FINE CHEMICALS) 02 juin 1988,<br>* le document en entier *<br>--- | 1, 6 | |
| D,A | US-A-4288636 (A. NISSEN ET AL.)<br>* colonne 3, ligne 31 - colonne 3, ligne 44; revendication 1 *<br>----- | 1 | |
| | | | DOMAINES TECHNIQUES RECHERCHES (Int. Cl.5 ) |
| | | | C07C |

Le présent rapport a été établi pour toutes les revendications

| Lieu de la recherche | Date d'achèvement de la recherche | Examinateur |
|---|---|---|
| BERLIN | 05 AOUT 1991 | PROBERT,C |

### CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)